# EUROPEAN PATENT SPECIFICATION

(11) **EP 2 167 019 B1**
(45) Date of publication and mention of the grant of the patent: **07.01.2015**
(21) Application number: 08781776.3
(22) Date of filing: 14.07.2008
(51) Int. Cl.: A61K 8/11, A61K 8/21, A61K 8/22, A61K 8/25, A61Q 11/00

(54) **COLOR STABLE PEROXIDE CONTAINING DENTIFRICE FORMULATIONS WITH DYE ENCAPSULATED SILICA SHELL NANOPARTICLES**
FARBSTABILE PEROXIDHALTIGE ZAHNPASTAFORMULIERUNGEN MIT FARBSTOFFVERKAPSELTEN NANOPARTIKELN MIT SILIKAHÜLLE
FORMULATIONS DE DENTIFRICE CONTENANT DU PEROXYDE À COULEUR STABLE AVEC NANOPARTICULES DE CARAPACE DE SILICE ENCAPSULÉES DANS UNE TEINTE

(30) Priority: 15.08.2007 US 839296
(43) Date of publication of application: 31.03.2010
(73) Proprietor: Colgate-Palmolive Company, New York, NY 10022 (US)
(72) Inventor: GU, Ben, East Brunswick, NJ 08816 (US); DIXIT, Nagaraj, Plainsboro, NJ 08536 (US); CUMMINS, Diane, Livingston, NJ 07039 (US); MASTERS, James, Gerard, Ringoes, NJ 08551 (US)
(74) Representative: Jenkins, Peter David
(86) International application number: PCT/US2008/069931
(87) International publication number: WO 2009/023393

(56) References cited:
- WO-A-01/80823
- WO-A-01/88540
- WO-A-2004/081222
- WO-A-2005/009604
- WO-A-2006/125661
- US-A- 5 683 679
- US-A- 6 074 629

## Description

### BACKGROUND OF THE INVENTION

Peroxide species are known to be reactive in the presence of a broad spectrum of toothpaste colorants and may decompose the color pigments rapidly. Additionally, the presence of fluoride ion in peroxide containing systems further enhances the rate of colorant decomposition, resulting not only in color bleaching, but also loss in peroxide stability due to the chemical (redox) reaction with the colorant. Several radical scavengers and reducing agents have been identified which reportedly slow, but do not substantially eliminate, incompatibility and chemical instability between a peroxide species and colorant such as FD&C Blue 1 and FD&C Yellow 5.

The art does not disclose a chemically and physiologically stable dentifrice gel comprising peroxide and fluoride ions with organic dyes to minimize color fading and degradation over an extended period of time both at ambient and at elevated temperature ranges.

US5,683,679 discloses oral compositions comprising a peroxide, a triphenylmethane class dye, a tartrazine class dye and a pharmaceutically acceptable carrier. This document discloses that a highly stable green dye system can be provided by containing a triphenylmethane class dye and a tartrazine class dye. Such a dye system does not fade in the presence of peroxides.

Consequently, there is a need for formulating all three components, fluoride ion, peroxide species and colorant (dye) in a single system with minimal loss and degradation in color of the dentifrice for a significant period of time at both ambient and elevated temperature ranges.

### BRIEF SUMMARY OF THE INVENTION

The invention includes a composition for minimizing color fading in an oral care composition that can include an organic dye-encapsulated silica shell nanoparticle matrix, a fluoride ion-providing compound and c) a physiologically stable peroxide species-providing compound (PSPC). The fluoride ion providing compound may be present in an amount of about 0.1% to about 1% by weight and the PSPC may be present in an amount of about 1% to about 20% by weight.

Also disclosed herein are methods of minimizing color fading in an oral dentifrice composition or mouthwash solution. Such methods include combining a silica shell encapsulated organic dye Nanoparticle and a formulation comprising peroxide species and fluoride ions, stabilizing the silica shell encapsulated dye nanoparticle, peroxide species, and fluoride ions in a dentifrice formulation or mouthwash solution, wherein the silica shell encapsulated dye nanoparticle provides color stability of the oral composition for at least three weeks at up to 49 °C.

### DETAILED DESCRIPTION OF THE INVENTION

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls.

The oral compositions of the present invention include products which are substantially liquid in character, such as a mouthwash or rinse, gel in character, paste in character and/or are used to coat or form films on oral care devices or implements, such as flosses or toothbrushes.

As used herein, the term "physiological stable" when referring to compounds means that the compounds do not substantially break down or otherwise become ineffective upon introduction to a subject prior to having a desired effect.

The present invention relates to compositions for minimizing color fading of an oral care composition and related methods. The composition of the invention includes an organic dye- encapsulated (silica shell nanoparticle matrix, fluoride ion-providing compound(s) and a peroxide species-providing compound ("PSPC"). The fluoride ion-providing compound may be physiologically stable and present in any amount desired; preferably about 0.1% to about 1% by weight. The PSPC may be a physiologically stable and is present in an amount of 1% to 20% by weight, for example about 1% to about 7% by weight, about 1% to about 10%, or about 1% to about 15%. Also described herein are compositions comprising about 20% or greater PSPC.

Various examples of methods of preparing silica shell nanoparticles encapsulating a dye molecule or other materials into the core have been reported. Reference is made, for example, to U.S. Patents 6,924,116 (Tan et al.); U.S. Patent 6, 548,264 (Tan et al.*),* and U.S. Patent 6,800,122 (Anderson et al.*)* each of which disclose methods of preparing nanoparticles having a core enveloped by silica (SiO₂) shell using a water-in-oil microemulsion method. Reference is also made to International Patent Publication (WO 2004/074504) (Wiesner et al.*),* which discloses preparing fluorescent silica based nanoparticles using a condensation method of a silica and dye precursors. Reference is also made to US Patent 6,913,825 (Ostafin et al.*),* which discloses a process for making mesoporous silicate nanoparticle coatings and hollow mesoporous silica nanoparticles by silicate crystal growth technique. The contents of each of these references are incorporated herein by reference in their entirety for description of preparation of silica shell encapsulated dye nanoparticle matrix, their characteristics and use in the present invention.

As described herein, organic dyes may include non-toxic, water insoluble organic dyes. The dyes used in the practice of the present invention are encapsulated within a silica shell nanoparticle matrix as described by any of the methods of (*e.g.*) Tan, Anderson, Ostafin and Wiesner *supra.* The dye encapsulated silica shell nanoparticle matrix is distributed uniformly throughout the dentifrice component and are food color additives presently certified under the Food Drug & Cosmetic Act for use in food and ingested drugs.

The dyes used in the compositions are FD&C Blue No. 1 (disodium salt of dibenzyldiethyldiaminotriphenylcarbinol trisulfonic acid anhydrite) and FD&C Yellow No. 5 (sodium salt of 4-p-sulfophenylazo-1-p-sulfophenyl-5-hydroxypyrazole-3 carboxylic acid). Also described herein are the dyes FD&C Red No.3 (sodium salt of tetraiodo-flurescein), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4- hydroxy-2-sulfoniumphenyl)-methylene}-[1-(N-ethyI-N-p-sulfobenzyl)-□ -3,5-cyclohexadienimine], FD&C Blue No. 2 (sodium salt of disulfonic acid of indigo tin) and mixtures thereof in various proportions. The concentration of the dye for the most effective result in the present invention is present in the dentifrice composition in an amount of about 0.05 percent to about 4 percent by weight.

The silica shell encapsulated dye of the composition of this invention is FD&C Blue No.1 or FD&C Yellow No. 5.

In an alternate exemplary embodiment, the organic dye encapsulated silica shell nanoparticle matrix of the composition of this invention exhibits a low surface area that survives osmotic pressure gradients resulting in no diffusion across the silica shell.

In the practice of the invention, the dye encapsulated silica shell nanoparticle matrix may be present in any amount. For example, the dye encapsulated silica shell nanoparticle matrix may be present in an amount of about 1% to about 4%, about 3% to about 10%, or about 5% to about 25% weight of the total composition.

Various types of compounds that provide peroxide species, referred to herein as "peroxide species providing compounds," or ("PSPC") may be used in this invention. In exemplary embodiments, PSPC include but are not limited to hydrogen peroxide, urea peroxide, calcium peroxide, persilicate, perphosphate, persulphate, perborate and percarbonate. Metal peroxides useful for the invention include peroxide containing compounds such as calcium peroxide, sodium peroxide, strontium peroxide, magnesium peroxide, and the salts of perborate, persilicate, perphosphate and percarbonate such as sodium perborate, potassium persilicate and sodium percarbonate.

Various types of compounds which provide fluoride ions, referred to herein as "fluoride ion providing compounds," may be used in this invention. In exemplary embodiments, the fluoride ion providing compound include but are not limited to sodium fluoride, potassium fluoride, calcium fluoride, magnesium fluoride, stannous fluoride, stannous monofluorophosphate, sodium monofluorophosphate and copper fluoride.

Additional water-miscible organic solvents may be present in the composition of invention. The water miscible solvent advantageously comprises or alternatively comprises, one or more alkyl glycol ethers, hereafter "glycol ethers." Glycol ethers are well known and include but are not limited to mono- or dialkyl ethers of polyols such as alkyl ethers of ethylene glycol. Exemplary glycol ether species useful in the dentifrice compositions include but are not limited to polyethylene glycol.

In an alternate exemplary embodiment the ethylene glycol of the composition of this invention includes polyethylene glycol in an amount at least about 5% by weight. In an alternate exemplary embodiment, the composition of this invention further includes phosphoric acid in an amount at least about 0.1% by weight. In some embodiments polishing agents may be present. Polishing agents useful for dentifrice compositions used in the practice of the present invention include but are not limited to silica, calcined alumina, sodium bicarbonate, calcium carbonate, dicalcium phosphate and calcium pyrophosphate may be included in the base dentifrice compositions used in the practice of the present invention. Visually clear dentifrice compositions are obtained by using polishing agents such as colloidal silica, such as those sold under the trade designation Zeodent 115 (Zeo 115) available from the Huber Corporation or alkali metal aluminosilicate complexes that have refractive indices close to the refractive indices of gelling agent-liquid (including water and/ or humectant) systems used in dentifrice compositions. In an alternate exemplary embodiment, the composition of this invention further includes silica abrasive in an amount of about 0.1% to about 0.5% by weight.

The dentifrice composition of the present invention may also contain a flavoring agent. Flavoring agents which are used in the practice of the present invention include but are not limited to essential oils as well as various flavoring aldehydes, esters, alcohols, and similar materials. Examples of the essential oils include oils of spearmint such as peppermint, wintergreen, sassafras, clove, sage, eucalyptus, majoram, cinnamon, lemon, lime, grapefruit, and orange. Also useful are such chemicals as menthol, carvone, and anethole.

In an alternate exemplary embodiment, the composition of this invention further includes a flavorant incorporated in the dentifrice or mouthwash composition at a concentration in an amount about 0.5% to about 2% by weight.

In an alternate exemplary embodiment, the composition of this invention further includes de-ionized water (DI) in an amount of about 30% to about 50% by weight.

Sweeteners may also be incorporated in the dentifrice composition of this invention. Sweeteners of the present invention include but are not limited to both natural and artificial sweeteners. Suitable sweetener include but are not limited to water soluble sweetening agents such as monosaccharides, disaccharides and polysaccharides such as xylose, ribose, glucose (dextrose), mannose, galactose, fructose (levulose), sucrose (sugar), maltose, water soluble artificial sweeteners such as the soluble saccharin salts,( *i.e.*), sodium or calcium saccharin salts, cyclamate salts dipeptide based sweeteners, such a L-aspartic acid derived sweeteners, such as L-aspartyl-L-phenylalaine methyl ester (aspartame).

In an alternate exemplary alternate embodiment the invention further includes a sweetening agent, wherein the sweetening agent is sodium saccharin in an amount of about 0.1% to about 0.4% by weight.

In the preparation of the base dentifrice in accordance with the present invention there is utilized an orally acceptable vehicle, including a water-phase with humectant which is preferably glycerin or sorbitol. In an exemplary embodiment, the composition of this invention further includes glycerin in an amount at least about 35% to about 45% by weight.

Examples of organic thickeners which may be used in the preparation of the peroxide gel in combination with the inorganic thickener include but are not limited to natural and synthetic gums such as carrageenan (Irish moss), xanthan gum and sodium carboxymethyl cellulose, starch, polyvinylpyrrolidone, hydroxyethylpropylcellulose, hydroxybutyl methyl cellulose, hydroxypropyl methyl cellulose, and hydroxyethyl cellulose, and carboxyvinyl polymers, commercially available under the trademarks "Carbopol 934, 940, 974 P" from B.F. Goodrich, these polymers consisting of colloidally water soluble polymers of polyacrylic acid crosslinked with of about 0.75% to about 2% of polyallyl sucrose or polyallyl pentaerythritol as a cross linking agent, often with molecular weights of about 4 to about 5 million or more.

In an exemplary embodiment the organic thickener such as Carbopol and or Xanthan may be incorporated in the dentifrice gel of the present invention in an amount of about 0.1% to about 5% by weight.

Various other materials may be incorporated into the dentifrice components of this invention. Non-limiting examples thereof include preservatives, potassium nitrate and potassium citrate and mixtures thereof, vitamins such as pantheon. These adjuvants are incorporated in the dentifrice components in amounts which do not substantially adversely affect the properties and characteristics desired, and are selected and used in proper amounts, depending upon the particular type of dentifrice component involved.

Also described herein is a method for minimizing color fading in a dentifrice or mouthwash solution for use in an oral cavity comprises:
a) providing a silica shell encapsulated organic dye nanoparticle,
b) combining the silica shell encapsulated dye nanoparticle with a formulation comprising peroxide species and fluoride ions,
c) stabilizing the silica shell encapsulated dye nanoparticle, peroxide species, and fluoride ions in a dentifrice formulation or mouthwash solution, and
wherein the silica shell encapsulated dye nanoparticle renders color stability of the dentifrice gel or mouthwash solution for at least three weeks at up to 49 °C.

### EXAMPLES

Exemplary embodiments of the present invention will be illustrated by reference to the following examples, which are included to exemplify, but not limit the scope of the present invention.

The following examples illustrate the test samples of dentifrice gels comprising dye encapsulated silica shell nanoparticle matrix and its effect on color stability and fading over a given time and temperature range.

Example 1: A dentifrice formulation, sample A, was prepared comprising a dentifrice gel with regular FD&C Blue 1 without peroxide species. The complete formulation is detailed under Table 1, sample A.

Example 2: A dentifrice formulation, sample B, was prepared comprising the current simple whitening peroxide gel containing FD&C Blue 1 dye and 5.71 % by weight of (35% hydrogen peroxide). The complete formulation is detailed under Table 1, sample B.

Example 3: A dentifrice formulation, sample C, was prepared comprising same composition as sample B but formulated with silica shell encapsulated FD&C Blue 1 dye.

Table 1: Table 1 illustrates the dentifrice formulations for three samples with and without peroxide and silica-shell encapsulated FD &C blue.

**TABLE 1: Dentifrice Test Samples Compositions**

| **Ingredients** | **Sample A (control) wt%** | **Sample B wt%** | **Sample C wt%** |
|---|---|---|---|
| Purified Water | 44.82 | 39.11 | 36.66 |
| Glycerin | 40.00 | 40.00 | 40.00 |
| Polyethylene Glycol 600 | 10.00 | 10.00 | 10.00 |
| COP Carbopol 974P | 2.10 | 2.10 | 2.10 |
| Xanthan | 0.40 | 0.40 | 0.40 |
| Sodium Saccharin | 0.25 | 0.25 | 0.25 |
| Sodium Fluoride | 0.49 | 0.49 | 0.49 |
| Silica Abrasive (Zeo 115) | 0.30 | 0.30 | 0.30 |
| Hydrogen Peroxide (35%) | --- | 5.71 | 5.71 |
| Phosphoric acid (85%) | 0.10 | 0.10 | 0.10 |
| FD&C Blue #1 solution (6.25%) | 0.36 | 0.36 | ---- |
| Silica Encapsulated FD&C Blue #1 (0.8%) | --- | --- | 2.81 |
| Mint Gel Flavor | 1.15 | 1.15 | 1.15 |
| Butylated Hydroxytoluene (BHT) | 0.03 | 0.03 | 0.03 |

A series of color stability tests were conducted to evaluate the effect of FD&C Blue #1 dye encapsulated silica shell nanoparticle matrix (0.8%) on a dentifrice gel formulation comprising peroxide species and fluoride ions.

Gel compositions having the same ingredients (except for different peroxide species and silica-shell encapsulated dye nanoparticles) were prepared as identified under Table 1. Examples 1-3 were studied and evaluated. Dentifrice gel samples were allowed to age at room temperature and at 49 °C for a period of at least three weeks. The color of the gel was recorded as an indication of long-term color stability of the dentifrice gels.

An international color system (CIELAB: measuring L*, a* and b*) was used to determine the color change of the aged gels. Delta E* represents the total color differences between room temperature aged sample and 49°C aged samples. The results of three aged samples are illustrated in Table 2.

**TABLE 2: Color Stability of Aged Gels**

| **Sample** | **L*** | **a*** | **b*** | **□ E** |
|---|---|---|---|---|
| Sample A (RT) | 35.18 | -16.75 | -34.80 | --- |
| Sample A (3 weeks at 120°C) | 37.50 | -19.47 | -34.27 | 3.61 |
| Sample B (RT) | 43.71 | -24.47 | -31.42 | --- |
| Sample B (3 weeks at 120°C) | 59.05 | -17.47 | -11.19 | 26.34 |
| Sample C (RT) | 41.32 | -12.31 | -23.82 | --- |
| Sample C (3 weeks at 120°C) | 41.70 | -14.80 | -19.71 | 7.52 |

As illustrated by the data in Table 2, the results clearly indicate that color loss (DE) due to a redox reaction between the peroxide species and colorant was minimized by using silica shell encapsulated FD&C Blue #1 dye during the aging cycle. Sample C (3 weeks at 120 °F) shows a change that is comparable to the control without encapsulated dye and without peroxide species. Sample B with peroxide species, but without encapsulated dye, however, indicates the greatest color loss and color degradation. Color for sample B was significantly faded due to oxidation of the dye and this oxidation occurs more rapidly with increasing temperature. But the sample C with peroxide and encapsulated silica shell dye indicates a color change comparable to that of the control sample A. Specifically, the improvement can also immediately be seen in the b* value (conversion from yellow to blue) and in the L* value (conversion from black to white).

Encapsulating an organic dye (colorant) in a silica based nanoparticle matrix in the presence of peroxide and fluoride ions for a dentifrice gel exhibits the most minimal color variation as a function of aging and exhibits similar color changes to the non peroxide gel (control) formulation. Without being bound by theory, this result is attributed to the increased shielding of the organic dye by the silica shell nanoparticle matrix against oxidative reaction with peroxide and fluoride species in the dentifrice gel. As such, the dentifrice composition not only improves and enhances the color stability of the dentifrice gel but also significantly improves consumers' perception of product aesthetics.

## Claims

1. A composition for minimizing color fading in an oral care composition comprising:
a) an organic dye-encapsulated silica shell nanoparticle matrix,
b) a physiologically stable fluoride ion-providing compound,
c) a physiologically stable peroxide species-providing compound (PSPC) in an amount 1% to 20% by weight, wherein the dye is FD&C Blue 1 or FD&C Yellow 5, and wherein the silica shell nanoparticle matrix is distributed uniformly throughout the oral care composition.

2. The composition of claim 1, wherein the dye-encapsulated silica shell nanoparticle matrix is present in an amount 1% to 4% by weight.

3. The composition of claim 1, wherein the PSPC is member chosen from hydrogen peroxide, urea peroxide, calcium peroxide, persilicate, perphosphate, persulphate, perborate and percarbonate.

4. The composition of claim 1, wherein the fluoride ion-providing compound is member chosen from sodium fluoride, potassium fluoride, calcium fluoride, magnesium fluoride, stannous fluoride, stannous monofluorophosphate, sodium monofluorophosphate and copper fluoride.

5. The composition of claim 1 further comprising polyethylene glycol in an amount of at least 5% by weight.

6. The composition of claim 5 further comprising phosphoric acid in an amount at least about 0.1% by weight, or further comprising silica abrasive in an amount of about 0.1% to about 0.5% by weight, or further comprising a flavoring agent, or further comprising water in an amount of 30% to 50% by weight, or further comprising a sweetening agent, wherein the sweetening agent is sodium saccharin.

7. The composition of claim 1 further comprises glycerin in an amount of at least 35% to 45% by weight.

8. A method for minimizing color fading in an oral care composition, comprising:
a) combining a silica shell encapsulated dye nanoparticle matrix with a formulation comprising peroxide species and fluoride ions, and
b) stabilizing the silica shell encapsulated dye nanoparticle matrix, peroxide species, and fluoride ions in a dentifrice formulation or mouthwash solution,
wherein the dye is FD&C Blue 1 or FD&C Yellow 5,
and wherein the silica shell encapsulated dye nanoparticle matrix is distributed uniformly throughout the oral care composition.

9. The method of claim 8, wherein the dye-encapsulated silica shell nanoparticle matrix is present in an amount of 1 % to 4% by weight.

10. The method of claim 8, wherein the composition further comprises polyethylene glycol in an amount of at least about 5% by weight.

11. The method of claim 10, wherein the composition further comprises silica abrasive in an amount 0.1% to 0.5% by weight.

## Patentansprüche

1. Eine Zusammensetzung zum Minimieren des Verblassens von Farben in einer Mundpflegezusammensetzung, die Folgendes umfasst:
a) eine organischen Farbstoff verkapselnde Nanopartikelmatrix mit Silikahülle,
b) eine physiologisch stabile, Fluoridionen liefernde Verbindung,
c) eine physiologisch stabile, Peroxid-Speziesliefernde Verbindung (PSPC: peroxide species-providing compound) im Umfang von 1 bis 20 Gewichtsprozent, wobei der Farbstoff FD&C Blue 1 (Blau 1) oder FD&C Yellow 5 (Gelb 5) und die Nanopartikelmatrix mit Silikahülle gleichmäßig in der gesamten Mundpflegezusammensetzung verteilt sind.

2. Die Anspruch 1 entsprechende Zusammensetzung, wobei die Farbstoff verkapselnde farbstoffverkapselnde Nanopartikelmatrix mit Silikahülle im Umfang von 1 bis 4 Gewichtsprozent vorhanden ist.

3. Die Anspruch 1 entsprechende Zusammensetzung, wobei die PSPC-Verbindung ein aus Wasserstoffperoxid, Ureaperoxid, Kalziumperoxid, Persilikat, Perphosphat, Persulfat, Perborat und Percarbonat ausgewähltes Mitglied ist.

4. Die Anspruch 1 entsprechende Zusammensetzung, wobei die Fluoridionen liefernde Verbindung ein aus Natriumfluorid, Kaliumfluorid, Calciumfluorid, Magnesiumfluorid, Zinnfluorid, Zinn-Monofluorophosphat, Natrium-Monofluorophosphat und Kupferfluorid ausgewähltes Mitglied ist.

5. Die Anspruch 1 entsprechende Zusammensetzung, die ferner Polyethylenglykol im Umfang von zumindest 5 Gewichtsprozent umfasst.

6. Die Anspruch 5 entsprechende Zusammensetzung, die ferner Phosphorsäure im Umfang von zumindest ca. 0,1 Gewichtsprozent oder ferner Silicaschleifmittel im Umfang von ca. 0,1 bis ca. 0,5 Gewichtsprozent oder ferner einen Aromastoff oder ferner Wasser im Umfang von 30 bis 50 Gewichtsprozent oder ferner ein Süßungsmittel umfasst, wobei das Süßungsmittel Saccharin-Natrium ist.

7. Die Anspruch 1 entsprechende Zusammensetzung, die ferner Glycerin im Umfang von zumindest 35 bis 45 Gewichtsprozent umfasst.

8. Ein Verfahren zum Minimieren des Verblassens von Farben in einer Mundpflegezusammensetzung, das Folgendes umfasst:
a) eine organischen Farbstoff verkapselnde Nanopartikelmatrix mit Silikahülle mit einer Formulierung kombinieren, die Peroxid-Spezies und Fluoridionen umfasst, und
b) die organischen Farbstoff verkapselnde Nanopartikelmatrix mit Silikahülle, Peroxid-Spezies und Fluoridionen in einer Zahnpastenformulierung oder Mundspüllösung stabilisieren,
wobei der Farbstoff FD&C Blue 1 (Blau 1) oder FD&C Yellow 5 (Gelb 5) ist,
und wobei die Farbstoff verkapselnde farbstoffverkapselnde Nanopartikelmatrix mit Silikahülle in der gesamten Mundpflegezusammensetzung gleichmäßig verteilt ist.

9. Das Anspruch 8 entsprechende Verfahren, wobei die Farbstoff verkapselnde farbstoffverkapselnde Nanopartikelmatrix mit Silikahülle in einem Umfang von 1 bis 4 Gewichtsprozent vorhanden ist.

10. Das Anspruch 8 entsprechende Verfahren, wobei die Zusammensetzung ferner Polyethylenglykol in einem Umfang von zumindest 5 Gewichtsprozent umfasst.

11. Das Anspruch 10 entsprechende Verfahren, wobei die Zusammensetzung ferner Silikaschleifmittel im Umfang von 0,1 bis 0,5 Gewichtsprozent umfasst.

## Revendications

1. Une composition pour minimiser la décoloration dans une composition de soins buccaux-dentaires comprenant :
(a) une matrice de nanoparticules de colorant organique encapsulées dans une enveloppe de silice,
(b) un composé fournissant des ions de fluorure physiologiquement stables
(c) un composé fournissant une espèce peroxyde physiologiquement stable (CFEP) dans une quantité de 1 % à 20 % en poids, dans lequel le colorant est FD&C Bleu 1 ou FD&C Jaune 5, et dans laquelle la matrice de nanoparticules dans une enveloppe de silice est distribuée de manière uniforme dans la totalité de la composition.

2. La composition de la revendication 1, dans laquelle la matrice de nanoparticules de colorant organique encapsulées dans une enveloppe de silice est présente dans une quantité de 1 % à 4 % en poids.

3. La composition de la revendication 1, dans laquelle le CFEP est un élément choisi parmi le peroxyde d'hydrogène, le peroxyde d'urée, le peroxyde de calcium, le persilicate, le perphosphate, le persulphate, le perborate et le percarbonate.

4. La composition de la revendication 1, dans laquelle le composé fournissant des ions de fluorure est un élément choisi parmi le fluorure de sodium, le fluorure de potassium, le fluorure de calcium, le fluorure de magnésium, le fluorure stanneux, le monofluorophosphate stanneux, le monofluorophosphate de sodium et le fluorure de cuivre.

5. La composition de la revendication 1 comprenant en outre du polyéthylène glycol dans une quantité d'au moins 5 % en poids.

6. La composition de la revendication 5 comprenant en outre de l'acide phosphorique dans une quantité d'au moins environ 0,1 % en poids, ou comprenant en outre de la silice abrasive dans une quantité d'environ 0,1 % à environ 0,5 % en poids, ou comprenant en outre un agent aromatisant, ou comprenant en outre de l'eau dans une quantité de 30 % à 50 % en poids, ou comprenant en outre un agent édulcorant, dans laquelle l'agent édulcorant est de la saccharine de sodium.

7. La composition de la revendication 1 comprenant en outre de la glycérine dans une quantité d'au moins 35 % à 45 % en poids.

8. Un procédé pour minimiser la décoloration dans une composition de soins buccaux-dentaires, comprenant :
a) la combinaison d'une matrice de nanoparticules de colorant organique encapsulées dans une enveloppe de silice avec une formulation comprenant une espèce de peroxyde et des ions de fluorure, et
b) la stabilisation de la matrice de nanoparticules de colorant organique encapsulées dans une enveloppe de silice, de l'espèce de peroxyde, et des ions de fluorure dans une formulation de dentifrice ou une solution de bain de bouche,
dans lequel le colorant est FD&C Bleu 1 ou FD&C Jaune 5, et dans laquelle la matrice de nanoparticules de colorant organique encapsulées dans une enveloppe de silice est distribuée de manière uniforme dans la totalité de la composition.

9. Le procédé de la revendication 8, dans lequel la matrice de nanoparticules de colorant organique encapsulées dans une enveloppe de silice est présente dans une quantité de 1 % à 4 % en poids.

10. Le procédé de la revendication 8, dans lequel la composition comprend en outre du polyéthylène glycol dans une quantité d'au moins environ 5 % en poids.

11. Le procédé de la revendication 10, dans lequel la composition comprend en outre de la silice abrasive dans une quantité de 0,1 % à 0,5 % en poids.
